Europäisches Patentamt

European Patent Office

Office européen des brevets

(11) Publication number: **0 474 870 A1**

## EUROPEAN PATENT APPLICATION
## published in accordance with Art. 158(3) EPC

(21) Application number: **91900073.7**

(22) Date of filing: **27.03.90**

(86) International application number: **PCT/SU90/00074**

(87) International publication number: **WO 91/14474 (03.10.91 91/23)**

(51) Int. Cl.5: **A61M 27/00, A61L 31/00**

(43) Date of publication of application: **18.03.92 Bulletin 92/12**

(84) Designated Contracting States: **BE CH DE FR GB IT LI SE**

(71) Applicant: **POLTAVSKY MEDITSINSKY STOMATOLOGICHESKY INSTITUT ul. Shevchenko, 23, Poltava 314000(SU)**

(72) Inventor: **MAZURIK, Sergei Mikhailovich ul. Lenina, 92-57 Poltava, 314022(SU)**
Inventor: **MAZURIK, Mikhail Fedotovich ul. Kotlyarevskogo, 16-7 Poltava, 314000(SU)**
Inventor: **TSYGANOVA, Nina Alexeevna ul. Lenina, 92-57 Poltava, 314022(SU)**

(74) Representative: **Lerwill, John A.A. Thornton & Co. Northumberland House 303-306 High Holborn London, WC1V 7LE(GB)**

(54) **DRAINAGE TUBE.**

(57) The invention relates to medical techniques. A drainage tube is made of an elastic material and is provided with perforations on its working end (2). The surface of the working end (2) is configurated and has protrusions and depressions, the surface area of the top of each protrusion being less than the cross-section of the pancreatic duct opening to prevent the closing of the pancreatic duct opening by the wall of the drainage tube when the latter is placed inside the common biliary duct. The working end (2) of the tube should preferably be formed of a grid-like interwinding of filaments (5). The drainage tube is intended for use in hospitals dealing with biliary duct surgery.

FIG. 1

## Field of the Invention

The present invention relates to medical technology, specifically to drainage tubes employed for transcapillary drainage of the common bile duct.

## Prior Art

Known in the present state of the art is a T-drain made of an elastic material (Latex), which consists of a transverse and longitudinal portions and is used for transcapillary drainage of the common bile duct (T-DRAIN, Cattell, code No. 2575, US, Inmed Corporation /2950 Pacific Drive/ Norcross, GA. 30071).

The transverse portion of the tube is placed intraoperatively in the common bile duct, and one of its ends is introduced into the duodenum via the major duodenal papilla in order to provide for the unobstructed outflow of bile. The wall of the transverse portion of the tube closes the lumen of the ostium of the pancreatic duct thus hindering the escape of the pancreatic juice into the common bile duct. The derangement of the free outflow of the pancreatic secretion leads to hypertension in the ducts of the pancreas, its tissue autolysis, and development of acute pancreatitis. This restricts the indications to transcapillary drainage despite the fact that this technique is in essence a method providing for most adequate drainage.

Known in the present state of the art is also a drain which is an elastic single-barrelled tube having perforations at its working end. The perforations are made in longitudinal depressions, and the tube itself has an outer covering with sieve-like fenestrations (PCT/EP 84/00220). The tube is used to draw secretions from the wound cavity. Owing to the presence of an outer covering, plugging of the inner tube perforations with mucus and secretion does not occur.

However, if such a drainage tube is placed in the common bile duct for transcapillary drainage, a bridge between the outer-covering fenestrations or a mucus-plugged fenestration may be found located against the ostium of the pancreatic duct. This would impair the escape of the secretion from the pancreatic duct with subsequent development of pancreatitis.

## Disclosure of the Invention

It is an object of the present invention to provide a drainage tube with the working end constructed in such a way as to exclude a possibility of obstructing the pancreatic duct ostium in any position of the drainage tube in the common bile duct.

The object of the invention is attained by that the drainage tube, made as an elastic single-barrelled tube having perforations at its working end, according to the invention, has its working end surface made uneven and consisting of projections and depressions, the apex area of each projection being smaller than the area of the ostium of the pancreatic duct.

It is expedient that the working end of the tube be made as a netlike intersection of fibers whose diameter is smaller than the ostium of the pancreatic duct, and the distance between the fibers is equal to, or larger than, the ostium of the pancreatic duct.

The fiber diameter may be chosen to be from 0.3 to 0.5 of the diameter of the ostium of the pancreatic duct.

A drainage tube made in accordance with the invention prevents the possibility of obstructing the pancreatic duct ostium with the tube surface during the tube insertion into the common bile duct for transcapillary drainage. The manufacturing costs of the drainage tube, according to the invention, insignificantly exceed the manufacturing costs of the tubes known in the present state of the art.

## Brief Description of the Drawings

In what follows the invention is illustrated with the description of specific exemplary embodiment thereof with refence to the accompanying drawings, wherein:

Fig. 1 presents a general view (isometry) of the drainage tube according to the invention;

Fig. 2 presents a section along the line II-II in Fig. 1;

Fig. 3 presents a diagrammatic representation of the position of the drainage tube according to the invention in the common bile duct during transcapillary drainage.

## Preferred Embodiment of the Invention

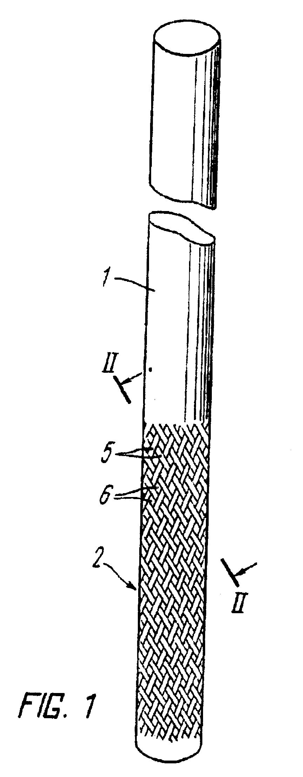

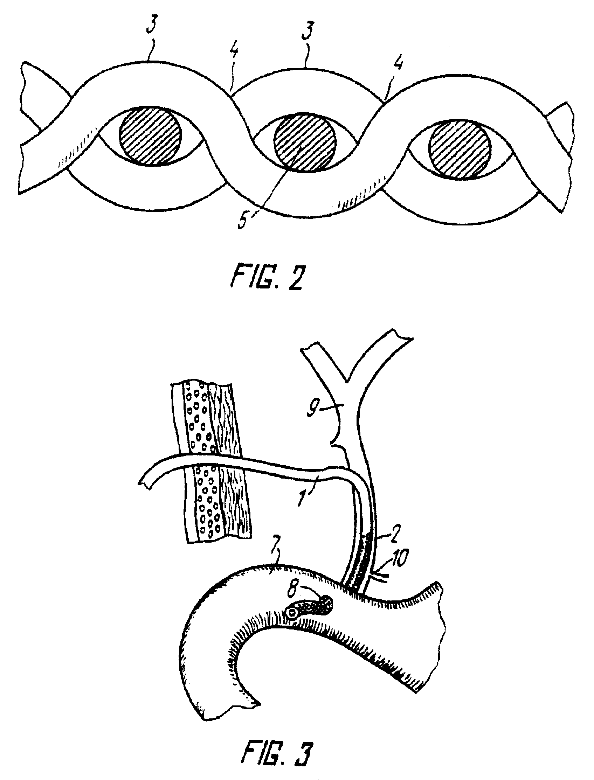

The drainage tube according to the invention, depicted in Figs 1, 2 is a single-barrelled tube 1 made of an elastic material, e.g. Latex, whose end 2 is the working end provided with perforations.

The outer surface of the working end 2 is made uneven and consists of proejections 3 and depressions 4, the surface area at the apex of each projection 3 being smaller than the diameter of the ostium of the pancreatic duct.

In the imbodiment of the invention described the projections 3 and depressions 4 on the working end 2 are made as a netlike intersection of fibers 5. The fibers 5 intersect at different levels and form meshes 6 which function as perforations. The diameter of the fibers 2 is 0.3-0.5 of the diameter of the lumen of the pancreatic duct and is commonly equal to 0.3-0.4 mm. The distance between the neighbouring fibers 5, i.e. the linear dimension of the sides of meshes 6, is equal to or exceeds the diameter of the lumen of the pancreatic duct and is commonly equal to 1.2-1.5 mm$^2$.

The area of the projection apices of the netlike structure is smaller than the area of the ostium of the pancreatic duct, since the diameter of the fibers 5 is chosen so as to be smaller than the diameter of the ostium of the pancreatic duct.

The drainage tube, according to the invention, is applied as follows.

During a surgical intervention, the working end 2 of the drainage tube 1 is passed through the choledochotomy wound into the duodenum 7 (Fig. 3) via the major duodenal papilla 8. The solid portion of the tube 1 is so positioned as to extend up to the place of entrance of the drainage tube 1 into the common bile duct 9 and somewhat further. This is necessary to prevent the fiber netlike structure from getting into the zone of exit of the tube 1 from the common bile duct 9 when the drainage tube 1 is being fixed in the common bile duct 9. Otherwise, leaking of bile into the free abdominal cavity may occur. The netlike portion of the drainage tube 1 passes by the ostium IO of the pancreatic duct and further via the major duodenal papilla 8 into the duodenum 7. The free outflow of the pancreatic secretion is ensured in any position of the drainage tube 1. This is attributed to the fact that no more than one fiber 5 (Fig. 2) can be situated in the region of the ostium IO of the pancreatic duct, which is ensured by the choice of the distance between the fibers 5. Intersection of the fibers 5 does not increase the surface area of the projection 3, since the fibers 5 are situated at different levels in the sites of intersection.

When passing through the meshes 6 (Fig. 1) formed by intersection of fibers 5, the pancreatic secretion will flow freely into the duodenum 7 (Fig. 3). The postoperative management of patients subjected to transcapillary drainage of the common bile duct using the proposed drainage tube according to the invention, has no specific features.

The proposed drainage tube is simple in operation and effective in application. It provides for free escape of the pancreatic juice in any position of the tube. Application of the proposed drainage tube allows for a broader use of the transcapillary drainage of the common bile duct with no risk of such severe complication as acute pancreatitis.

Industrial Applicability

The proposed invention may be used in inpatient settings specialized at surgery of the biliary tract.

**Claims**

1. A drainage tube made of an elastic material and having perforations at its working end (2), CHARACTERIZED in that the surface of the working end (2) is made uneven and comprises projections (3) and depressions (4), each projection (3) having the area at the apex thereof smaller than the area of the ostium (IO) of the pancreatic duct.

2. A drainage tube as claimed in Claim 1, CHARACTERIZED in that the working end (2) is formed by a netlike intersection of fibers (5) whose diameter is smaller than, and the distance between which is at least equal to, the diameter of the ostium (IO) of the pancreatic duct.

3. A drainage tube as claimed in Claim 2, CHARACTERIZED in that the diameter of the fibers (5) is from 0.3 to 0.5 of the diameter of the ostium (IO) of the pancreatic duct.

FIG. 1

FIG. 2

FIG. 3

# INTERNATIONAL SEARCH REPORT

International Application No PCT/SU 90/00074

| I. CLASSIFICATION OF SUBJECT MATTER (if several classification symbols apply, indicate all) * |
|---|

According to International Patent Classification (IPC) or to both National Classification and IPC

IPC$^5$   A61M 27/00, A61L 31/00

## II. FIELDS SEARCHED

Minimum Documentation Searched [7]

| Classification System | Classification Symbols |
|---|---|
| IPC$^5$ | A61M 27/00, A61L 31/00 |

Documentation Searched other than Minimum Documentation
to the Extent that such Documents are Included in the Fields Searched [8]

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| A | CH, A5, 484679 (HARALD WILKE) 13 March 1970 (13.03.70) the claims,the drawing | 1 |
| A | DE, A1, 3325920 (STERIMED GEZELLSCHAFT FÜR MEDIZINISCHEN BEDARF MBH) 7 February 1985 (7.02.85) the claims,figures 1,21 | 1,2,3 |
| A | SU, A1, 1437035 (Voroshilovgradtsky medical institut) 15 November 1988 (15.11.88) the claims, fig. 1, 2 | 1,2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 19 February 1991 (19.02.91) | 4 March 1991 (04.03.91) |

| International Searching Authority | Signature of Authorized Officer |
|---|---|
| ISA/SU | |

Form PCT/ISA/210 (second sheet) (January 1985)